# NEUE EUROPÄISCHE PATENTSCHRIFT

(11) **EP 0 340 592 B2**
(45) Veröffentlichungstag und Bekanntmachung des Hinweises auf die Entscheidung über den Einspruch: **18.03.1998**
(45) Hinweis auf die Patenterteilung: 09.03.1994
(21) Anmeldenummer: 89107373.6
(22) Anmeldetag: 24.04.1989
(51) Int. Cl.: A61K 7/06

(54) **Haarbehandlungsmittel mit natürlichen Inhaltsstoffen**
Hair treatment composition with natural ingredients
Composition pour le traitement des cheveux contenant des composés naturels

(30) Priorität: 02.05.1988 DE 3814839
(43) Veröffentlichungstag der Anmeldung: 08.11.1989
(73) Patentinhaber: Henkel Kommanditgesellschaft auf Aktien, 40191 Düsseldorf (DE)
(72) Erfinder: Müller, Reinhard, Dr., D-4018 Langenfeld (DE); Höffkes, Horst, Dr., D-4000 Düsseldorf-Hellerhof (DE); Seidel, Kurt, D-4000 Düsseldorf 13 (DE); Wisotzki, Klaus-Dieter, Dr., D-4006 Erkrath 2 (DE)

(56) Entgegenhaltungen:
- EP-A- 0 019 301
- WO-A-80/01144
- DE-C- 715 803
- FR-A- 1 439 040
- US-A- 4 115 313
- US-A- 4 832 872
- PATENT ABSTRACTS OF JAPAN, Band 8, Nr. 184 (C-239)[1621], 23. August 1984;& JP-A-59 78 113 (SHISEIDO) 04-05-1984
- H. JANISTIJN: "Handbuch der Kosmetika und Riechstoffe", Band 3: "DieKörperpflegemittel", Auflage 2, 1973, Hüthig Verlag, Heidelberg,
- PATENT ABSTRACTS OF JAPAN, Band 11, Nr. 369 (C-461)[2816], 2. Dezember 1987;& JP-A-62 142 108 (AIKUSU LAB. SANGYO K.K.) 25-06-1987
- Grundlagen und Rezepturen der Kosmetika, Hüthig Verlag, 1979, K. Schrager, S. 471-477

## Beschreibung

Die Erfindung betrifft die Verwendung von natürlichen Phospholipiden und Säuren als Wirkstoffkombination für Haarbehandlungsmittel in Form einer wäßrigen Emulsion.

Das menschliche Haupthaar wird heute einer Vielzahl von Behandlungen ausgesetzt. Dazu gehören die Reinigung mit Shampoos, Duschbädern und Badepräparaten, aber auch kosmetische Prozeduren wie das Bleichen, Färben oder Verformen. Als Folge dieser Prozesse kann es zu ungewünschten Beeinträchtigungen der Haarstruktur kommen. Diese Beeinträchtigungen zeigen sich u. a. in einer schlechteren Naß- und Trockenkämmbarkeit der Haare. Des weiteren neigt das Haar zur elektrostatischen Aufladung, die sich negativ auf den Sitz der Frisur auswirkt.

Eine bekannte Möglichkeit zur Überwindung dieser Mißstände ist es, die Haare einer Nachbehandlung mit entsprechenden Wirkstoffen, zumeist kationischen Tensiden in Kombination mit weiteren Substanzen, zu unterziehen. Solche Haarnachbehandlungsmittel, die üblicherweise in Form von Haarwässern oder cremigen Emulsionen konfektioniert werden, werden beispielsweise in der DE-OS 3417646, in der japanischen Patentschrift 59/78113 und in der russischen Patentschrift 1090401 beschrieben.

Die genannten Haarbehandlungsmittel weisen jedoch Nachteile auf.

Es ist bekannt, daß sich kationische Tenside nur für die Behandlung von trockenem Haar gut eignen; ihre Anwendung bei rasch nachfettendem Haar ist dagegen problematisch, da sie die natürliche Nachfettung der Haare verstärken. Da kationische Tenside häufig eine unbefriedigende Haut- und Schleimhautverträglichkeit aufweisen, können sie zudem nur in begrenzten Mengen in Haarbehandlungsmitteln eingesetzt werden.

Außerdem sind die üblicherweise als kationische Tenside eingesetzten quartären Ammoniumverbindungen nur unzureichend biologisch abbaubar, so daß ihr Einsatz aus ökologischen Gründen möglichst vermieden werden sollte.

Es bestand daher die Aufgabe, Wirkstoffkombinationen für Haarbehandlungsmittel zu finden, die die Naß- und Trockenkämmbarkeit von Haaren verbessern und die statische Aufladbarkeit der Haare reduzieren, ohne die aufgeführten Nachteile aufzuweisen.

Beim Konsumenten besteht aufgrund der anhaltenden Chemiediskussion jedoch verstärkt der Wunsch nach Körperpflegemitteln, die möglichst nur aus natürlichen Inhaltsstoffen bestehen, d.h. Stoffen, die in der belebten oder unbelebten Natur und sogar im menschlichen Organismus auftreten.

Der Ersatz von synthetischen Komponenten durch natürliche Substanzen darf aber gerade bei Produkten für die Körperpflege nicht zu Lasten des optischen Erscheinungsbildes und der sensorischen Eigenschaften des Produktes gehen. Es bestand daher ebenfalls die Aufgabe, ein Haarbehandlungsmittel auf Basis dieser Wirkstoffkombinationen bereitzustellen, das in seiner Konfektionierungsform den Verbraucheransprüchen genügt.

Es wurde nun überraschend gefunden, daß die genannten Forderungen erfüllt werden können durch Verwendung einer Mischung von Phospholipiden natürlichen Ursprungs mit anorganischen und/oder organischen Säuren als Wirkstoffkombination in einer sauren wäßrigen Haarbehandlungsemulsion zur Verbesserung der Naß- und Trockenkämmbarkeit von Haaren und zur Reduzierung ihrer elektrostatischen Aufladbarkeit, wenn die Emulsion einen pH-Wert von 2 bis 3,5 aufweist und dadurch gekennzeichnet ist, daß die organischen Säuren ausgewählt sind aus der Gruppe, die Essigsäure, Milchsäure, Weinsäure, Zitronensäure, Äpfelsäure, Ascorbinsäure und Gluconsäure umfaßt. Dabei ist es möglich, gewünschtenfalls auf den Einsatz von Fettalkoholen, ethoxylierten Tensiden und anderen synthetischen Wirkstoffen sowie die üblichen Wachs- und Ölkomponenten zu verzichten. Es werden daher solche Mischungen bevorzugt, die ausschließlich natürliche, d.h. in der Natur vorkommende, Komponenten enthalten.

Zur Überwindung der elektrostatischen Aufladung des Haares sind sog. Neutralisierungs-Shampoos (z.B. aus Cosmetics & Toiletries, Vol 98, May 1983, S. 66) bekannt, die als Wirkstoffe neben großen Mengen synthetischer Tenside Phosphatester, Zitronensäure und Aminosäuren enthalten.

In der japanischen Patentschrift 61/238718 werden mikrobizide und Schuppen-bekämpfende Haarbehandlungsmittel beschrieben, die in einer wäßrig-ethanolischen Lösung neben einem synthetischen mikrobiziden Wirkstoff als weitere Komponenten natürlich vorkommende Phosphorverbindungen und/oder organische Säuren und/oder deren Salze enthalten können.

Aus der FR-A-1439040 sind Haarbehandlungsmittel bekannt, die Lecithin und anorganische oder organische Säuren enthalten und auf einen pH-Wert von 5, den pH-Wert der menschlichen Haut, eingestellt sind. US-A-4115313 offenbart Haarbehandlungsmittel, die Lecithin in Kombination mit Gallensäuren, Fettsäuren und Aminosäuren enthalten. In den "Patent Abstracts of Japan", Band 8, Nr. 184 (C-239)(1621) werden Haarbehandlungsmittel beschrieben, die Lecithin sowie C₁₂₋₂₂-Fettsäuren enthalten.

Keiner dieser Schriften sind jedoch Hinweise auf die erfindungsgemäßen Wirkstoffkombinationen aus Phospholipiden natürlichen Ursprungs und anorganischen und/oder organischen Säuren oder deren mögliche Wirkung in stärker sauren Emulsionen zu entnehmen.

Im Rahmen der Erfindung ist die Verwendung von Haarbehandlungsemulsionen bevorzugt, die 0,1 - 20 Gew.-%, insbesondere 1 - 3 Gew.-%, eines Phospholipids natürlichen Ursprungs, 0,1 - 20 Gew.-%, insbesondere 1 - 3 Gew.-%, einer anorganischen Säure und/oder einer organischen Säure aus der genannten Gruppe sowie 0,01 - 2 Gew.-%, insbesondere 0,05 - 0,5 Gew.-%, eines zusätzlichen Emulgators enthalten.

Es ist weiterhin bevorzugt, daß die Komponenten der Wirkstoffkombination, d.h. das Phospholipid und die anorganische und/oder die organische Säure, in einem Gewichtsverhältnis von 2 : 1 bis 1 : 2 in der Haarbehandlungsemulsion vorliegen.

Geeignete natürliche Phospholipide sind vor allem Lecithine, wie beispielsweise das Ei-Lecithin und das Soja-Lecithin. Es ist aber auch möglich, als Phospholipid Kephaline einzusetzen.

Als Säurekomponente der Wirkstoffkombination können sowohl anorganische als auch organische Säuren verwendet werden. Es ist bevorzugt, solche Säuren zu verwenden, die auch in der belebten und unbelebten Natur vorkommen.

Geeignete anorganischen Säuren sind z. B. Phosphorsäure, Schwefelsäure und Salzsäure.

Erfindungsgemäß verwendbare organische Säuren sind Essigsäure, Milchsäure, Weinsäure, Zitronensäure, Äpfelsäure, Ascorbinsäure und Gluconsäure. Zitronensäure und Ascorbinsäure sind als Säurekomponenten der Wirkstoffkombination besonders geeignet.

Die sauren Haarbehandlungsemulsionen weisen einen pH-Wert von 2 bis 3,5 auf. Besonders gute Eigenschaften zeigen Emulsionen, die einen pH-Wert zwischen 2,5 und 3,5 aufweisen. Die pH-Wert-Einstellung wird durch Art und Menge der verwendeten Säuren und gegebenenfalls durch zusätzliche Zugabe einer Base, z.B. eines Alkalimetallhydroxids oder eines Amins wie Triethanolamin, erzielt. Durch die Kombination von Säure und Base kann eine Pufferung, d.h. eines Stabilisierung des pH-Wertes der Emulsion im eingestellten pH-Bereich erreicht werden.

Da die verwendeten Phospholipide im Gegensatz zu den Säuren nicht wasserlöslich sind, kann die Wirkstoffkombination nicht in Form einer klaren wäßrigen Lösung konfektioniert werden. Die Verwendung von Gemischen aus Wasser und wasserlöslichen Alkoholen, wie beispielsweise Ethanol oder Isopropanol, als gemeinsames Lösungsmittel für die Wirkstoffkombination ist mit dem Nachteil verbunden, daß die zur Erzielung einer klaren Lösung notwendigen hohen Alkoholmengen zu Irritationen der Kopfhaut führen können.

Es hat sich jedoch gezeigt, daß die emulgierenden Eigenschaften der verwendeten Phospholipide üblicherweise ausreichen, um die Wirkstoffkombination in Form einer wäßrigen Emulsion zu konfektionieren.

Gleichwohl ist es möglich, die Stabilität der Emulsion, vor allem im Hinblick auf eine Verlängerung der möglichen Lagerdauer, durch Zusatz weiterer Emulgatoren zu erhöhen. Durch diese Emulgatoren läßt sich auch die Konsistenz der Emulsion in gewünschter Weise beeinflussen. Bezüglich der genannten Emulgatoren ist es wiederum bevorzugt, solche Verbindungen einzusetzen, die auch in der belebten Natur gefunden werden.

Bekannte natürliche Emulgatoren sind beispielsweise Gallensäuren, von denen insbesondere die Cholsäure, die Desoxycholsäure und die Lithocholsäure sowie als Derivat der Cholsäure die Taurocholsäure zu nennen sind. Gerbsäure, Abietinsäure und Saponine sind weitere geeignete Emulgatoren.

Es ist möglich, die genannten Säuren als freie Säuren einzusetzen. Man kann jedoch auch die entsprechenden Alkalisalze, insbesondere das Lithium-, Natrium- oder Kaliumsalz, oder das Ammoniumsalz einsetzen. Diese Salze können mit den bekannten Verfahren durch Umsetzung der entsprechenden Säuren mit den jeweiligen Alkalicarbonaten, Alkalihydrogencarbonaten oder Ammoniak erhalten werden. In der Regel weisen die Salze bessere Emulgatoreigenschaften als die freien Säuren auf. Innerhalb der Alkalisalze nimmt das Emulgiervermögen in der Reihe Lithium, Natrium, Kalium zu. Es ist daher besonders bevorzugt, Kaliumsalze, insbesondere das Kaliumsalz der Cholsäure, als zusätzlichen Emulgator einzusetzen.

Es ist dem Fachmann bekannt, daß die Konsistenz einer wäßrigen Emulsion durch Zugabe sogenannter Verdickungsmittel gezielt zu beeinflussen ist. Es kann daher vorgesehen sein, daß die erfindungsgemäßen Emulsionen 0,1 bis 10 Gew.-%, insbesondere 0,5 bis 5 Gew.-% eines Verdickungsmittels enthalten, um eine für die Anwendung geeignete Viskosität zu erzielen. Besonders vorteilhaft für sogenannte Haarspülungen sind Emulsionen mit Viskositäten im Bereich von 2500 bis 4000 mPas, insbesondere im Bereich von 3000 bis 3500 mPas, bei 20 °C. Für Haarbehandlungsemulsionen, die in Form sogenannter Haarkuren angeboten werden, sind dagegen Emulsionen mit Viskositäten zwischen 10 000 und 100 000 mPas üblich.

Es ist dabei wiederum bevorzugt, Verdickungsmittel zu verwenden, die in der belebten oder unbelebten Natur auftreten. Solche Verdickungsmittel sind beispielweise Polysaccharide, insbesondere Xanthan-Gum, Guar-Gum, Agar-Agar, Alginate, Johannisbrot-Kernmehl und Pektine.

Die saure Einstellung der Haarbehandlungsemulsion wirkt sich auch positiv auf ihre Haltbarkeit aus. Des weiteren zeigen einige der als Wirkstoffkomponente verwendeten Säuren bereits konservierende Eigenschaften, die für den üblichen Gebrauch ausreichend sind. Es kann jedoch im Rahmen der Erfindung vorgesehen sein, der Emulsion zusätzlich weitere Konservierungsmittel zuzumischen. Geeignete Konservierungsmittel sind beispielsweise Salicylsäure, Ameisensäure, Propionsäure, Benzoesäure, Sorbinsäure, Zimtsäure, Menthol, Thymol, Eugenol und Lemon Grass Extrakt. Diese Konservierungsmittel werden üblicherweise in Mengen von 0,1 bis 2 Gew.-% in der Haarbehandlungsemulsion eingesetzt. Die Verwendung von Benzoesäure als Konservierungsmittel ist besonders bevorzugt.

Die erfindungsgemäßen Emulsionen können weiterhin die üblichen, dem Fachmann bekannten Bestandteile von Haarbehandlungsmitteln in Mengen von maximal 5 Gew.-% enthalten. Solche Bestandteile sind beispielsweise Farbstoffe, Duftstoffe, Tenside, Antioxidantien, Lichtschutzmittel und haarkosmetische Wirkstoffe wie Vitamine, Pflanzenextrakte, Balsame, Antischuppenwirkstoffe oder Sebostatika sowie Ölkomponenten, bevorzugt natürliche pflanzliche und tierische Öle und Fette, und Wachse.

Die nachfolgende Beispiele sollen den Erfindungsgegenstand näher erläutern, ohne ihn darauf zu beschränken.

### Beispiele

Die in der nachstehenden Tabelle I aufgeführten Haarbehandlungs-Emulsionen wurden wie folgt hergestellt:
Phospholipid, Säure und zusätzlicher Emulgator wurden bei 70-80 °C aufgeschmolzen. Sodann wurde unter kräftigem Rühren das Konservierungsmittel, gelöst in ca. 65 Gewichtsteilen heißem Wasser, hinzugegeben. Nach dem Abkühlen der Mischung unter starkem Rühren auf ca. 45 °C erfolgte die Zugabe des Verdickungsmittels in Form einer wäßrigen Quellung mit dem restlichen Wasser. Es entstanden jeweils hellgelbe, feine Emulsionen. Im Fall der Beispiele 1 und 2 wurden diese Emulsionen durch Zugabe einer entsprechenden Menge Triethanolamin auf den gewünschten pH-Wert eingestellt.

Die angegebenen Viskositätswerte wurden bestimmt mit einem Viskosimeter Brookfield RVF, Spindel 4, bei 20 Umdrehungen pro Minute.

| Bestandteile (Gew.-Teile) | 1 | 2 | 3 | 4 | 5 | 6 | 7 | 8 | 9 |
|---|---|---|---|---|---|---|---|---|---|
| Phospholipid | | | | | | | | | |
| Sojalecithin | 1,7 | 1,7 | 2,5 | 4,0 | 1,7 | - | 1,7 | 1,68 | 2,5 |
| Eilecithin | - | - | - | - | - | 1,7 | - | - | - |

| Säure | | | | | | | | | |
|---|---|---|---|---|---|---|---|---|---|
| Zitronensäure | 1,7 | 1,7 | 1,7 | 4,0 | 2,4 | 1,7 | - | - | - |
| Milchsäure | - | - | - | - | - | - | - | - | 1,7 |
| Äpfelsäure | - | - | - | - | - | - | 1,7 | - | - |
| Phosphorsäure | - | - | - | - | _ | _ | _ | 1,68 | - |

| Emulgator | | | | | | | | | |
|---|---|---|---|---|---|---|---|---|---|
| Cholsäure-Na-Salz | 0,1 | 0,1 | - | - | - | 0,1 | - | 0,1 | - |
| Cholsäure-K-Salz | - | - | - | - | 0,1 | - | - | - | - |
| Cholsäure | - | - | - | - | - | - | - | - | 0,5 |
| Saponin | - | - | 0,5 | - | - | - | - | - | - |
| Taurocholsäure-Na-Salz | - | - | - | 0,2 | - | - | - | - | - |
| Desoxycholsäure | - | - | - | - | - | _ | 0,1 | - | - |

| Verdickungsmittel | | | | | | | | | |
|---|---|---|---|---|---|---|---|---|---|
| Xanthan-Gum | 1,0 | 1,0 | 1,0 | 1,0 | - | 1,0 | 1,0 | 1,0 | 1,0 |
| Na-alginat | - | - | - | - | 3,5 | - | - | - | - |

| Konservierungsmittel | | | | | | | | | |
|---|---|---|---|---|---|---|---|---|---|
| Benzoesäure | 0,5 | 0,5 | 0,4 | - | 0,5 | 0,3 | 0,3 | 0,5 | 0,4 |
| Sorbinsäure | - | - | - | 0,4 | - | - | - | - | - |
| Wasser | 95 | 95 | 93,9 | 90,4 | 90,2 | 95,2 | 95,2 | 95,04 | 93,9 |
| Triethanolamin | ad pH 5,9 | ad pH 4,1 | - | - | - | - | - | - | - |
| Glucose | - | - | - | - | 2,0 | - | - | - | - |
| pH-Wert | 5,9 | 4,1 | 3,2 | 3,0 | 3,1 | 2,4 | 2,5 | 2,0 | 2,9 |
| Viskosität (mPas) bei 20°C | - | - | 3000 | 3500 | 3950 | 3500 | 3650 | 3850 | 4600 |

Die mit Emulsionen gemäß Beispiel 1 (nicht erfindungsgemäß) behandelten Haare zeigten lediglich eine befriedigende Naß- und Trockenkämmbarkeit sowie eine leicht reduzierte elektrostatische Aufladbarkeit.

Eine gute Naß- und Trockenkämmbarkeit sowie eine deutlich reduzierte elektrostatische Aufladbarkeit wurde durch Behandlung der Haare mit Emulsionen gemäß Beispiel 2 erzielt. Die Behandlung von Haaren mit Emulsionen gemäß Beispielen 3 - 9 führte zu einer sehr guten Naß- und Trockenkämmbarkeit. Des weiteren wiesen die so behandelten Haare praktisch keine elektrostatische Aufladbarkeit mehr auf.

## Patentansprüche

1. Verwendung einer Mischung von Phospholipiden natürlichen Ursprungs mit anorganischen und/oder organischen Säuren als Wirkstoffkombination in einer sauren wäßrigen Haarbehandlungs-Emulsion zur Verbesserung der Naß- und Trockenkämmbarkeit von Haaren und zur Reduzierung ihrer elektrostatischen Aufladbarkeit, dadurch gekennzeichnet, daß
(a) die organischen Säuren ausgewählt sind aus der Gruppe, die Essigsäure, Milchsäure, Weinsäure, Zitronensäure, Äpfelsäure, Ascorbinsäure und Gluconsäure umfaßt, und
(b) die Emulsion einen pH-Wert von 2 bis 3,5 aufweist.

2. Verwendung nach Anspruch 1 in einer wäßrigen Emulsion, die zusätzliche Emulgatoren, bevorzugt natürlichen Ursprungs, enthält.

3. Verwendung nach einem der Ansprüche 1 oder 2 in einer wäßrigen Emulsion, die einen pH-Wert von 2,5 bis 3,5 aufweist.

4. Zur Verwendung nach einem der Ansprüche 1 bis 3 geeignete Haarbehandlungs-Emulsion, enthaltend eine Mischung von Phospholipiden natürlichen Ursprungs mit anorganischen und/oder organischen Säuren als Wirkstoffkombination sowie zusätzliche Emulgatoren, dadurch gekennzeichnet, daß
(a) die organischen Säuren ausgewählt sind aus der Gruppe, die Essigsäure, Milchsäure, Weinsäure, Zitronensäure, Äpfelsäure, Ascorbinsäure und Gluconsäure umfaßt, und
(b) die Emulsion einen pH-Wert von 2 bis 3,5 aufweist.

5. Haarbehandlungs-Emulsion nach Anspruch 4, dadurch gekennzeichnet, daß die Emulsion
- 0,1 bis 20 Gew.-%, bevorzugt 1 bis 3 Gew.-%, eines Phospholipids natürlichen Ursprungs,
- 0,1 bis 20 Gew.-%, bevorzugt 1 bis 3 Gew.-%, einer anorganischen Säure und/oder einer organischen Säure sowie
- 0,01 bis 2 Gew.-%, bevorzugt 0,05 bis 0,5 Gew.-%, eines zusätzlichen Emulgators, bevorzugt natürlichen Ursprungs, enthält.

6. Haarbehandlungs-Emulsion nach einem der Ansprüche 4 oder 5, dadurch gekennzeichnet, daß die Emulsion einen pH-Wert von 2,5 bis 3,5 aufweist.

7. Haarbehandlungs-Emulsion nach einem der Ansprüche 4 bis 6, dadurch gekennzeichnet, daß die Emulsion das Phospholipid und die anorganische und/oder organische Säure in einem Gewichtsverhältnis von 2:1 bis 1:2 enthält.

8. Haarbehandlungs-Emulsion nach einem der Ansprüche 4 bis 7, dadurch gekennzeichnet, daß das Phospholipid ein Lecithin, insbesondere Ei-Lecithin oder Soja-Lecithin, und/oder ein Kephalin ist.

9. Haarbehandlungs-Emulsion nach einem der Ansprüche 4 bis 8, dadurch gekennzeichnet, daß die anorganische Säure Phosphorsäure, Schwefelsäure und/oder Salzsäure ist.

10. Haarbehandlungs-Emulsion nach einem der Ansprüche 4 bis 8, dadurch gekennzeichnet, daß als organische Säure Zitronensäure oder Ascorbinsäure verwendet wird.

11. Haarbehandlungs-Emulsion nach einem der Ansprüche 4 bis 10, dadurch gekennzeichnet, daß der zusätzliche Emulgator Cholsäure, Lithocholsäure, Desoxycholsäure, Taurocholsäure, Gerbsäure, Abietinsäure, insbesondere in Form ihrer Alkalimetall- oder Ammoniumsalze, und/oder ein Saponin ist.

12. Haarbehandlungs-Emulsion nach einem der Ansprüche 4 bis 11, dadurch gekennzeichnet, daß der zusätzliche Emulgator ein Salz der Cholsäure, insbesondere das Kaliumsalz der Cholsäure, ist.

13. Haarbehandlungs-Emulsion nach einem der Ansprüche 4 bis 12, dadurch gekennzeichnet, daß die Emulsion 0,1 bis 10 Gew.-%, bevorzugt 0,5 bis 5 Gew.-%, eines Verdickungsmittels enthält und dabei eine cremige Beschaffenheit aufweist.

14. Haarbehandlungs-Emulsion nach Anspruch 13, dadurch gekennzeichnet, daß die Emulsion als Verdickungsmittel Polysaccharide, insbesondere Xanthan-Gum, Guar-Gum, Agar-Agar, Alginate, Johannisbrot-Kernmehl und/oder Pektine, enthält.

15. Haarbehandlungs-Emulsion nach einem der Ansprüche 4 bis 14, dadurch gekennzeichnet, daß die Emulsion zusätzliche Konservierungsmittel enthält.

16. Haarbehandlungs-Emulsion nach Anspruch 15, dadurch gekennzeichnet, daß die zusätzlichen Konservierungsmittel ausgewählt sind aus Salicylsäure, Ameisensäure, Propionsäure, Benzoesäure, Sorbinsäure, Zimtsäure, Menthol, Thymol, Eugenol und Lemon Grass Extrakt, wobei der Benzoesäure besondere Bedeutung zukommt.

## Claims

1. The use of a mixture of phospholipids of natural origin with inorganic and/or organic acids as an active-substance combination in an acidic aqueous hair treatment emulsion for improving the wet and dry combability and reducing the electrostatic charging of hair, characterized in that
(a) the organic acids are selected from the group consisting of acetic acid, lactic acid, tartaric acid, citric acid, maleic acid, ascorbic acid and gluconic acid and
(b) the emulsion has a pH value of 2 to 3.5.

2. The use claimed in claim 1 in an aqueous emulsion containing additional emulsifiers, preferably of natural origin.

3. The use claimed in claim 1 or 2 in an aqueous emulsion having a pH value in the range from 2.5 to 3.5.

4. A hair treatment emulsion suitable for the use claimed in any of claims 1 to 3, containing a mixture of phospholipids of natural origin with inorganic and/or organic acids as active-substance combination and additional emulsifiers, characterized in that
(a) the organic acids are selected from the group consisting of acetic acid, lactic acid, tartaric acid, citric acid, maleic acid, ascorbic acid and gluconic acid and
(b) the emulsion has a pH value of 2 to 3.5.

5. A hair treatment emulsion as claimed in claim 4, characterized in that the emulsion contains
- from 0.1 to 20% by weight and preferably from 1 to 3% by weight of a phospholipid of natural origin,
- from 0.1 to 20% by weight and preferably from 1 to 3% by weight of an inorganic acid and/or an organic acid and
- from 0.01 to 2% by weight and preferably from 0.05 to 0.5% by weight of an additional emulsifier, preferably of natural origin.

6. A hair treatment emulsion as claimed in claim 4 or 5, characterized in that the emulsion has a pH value in the range from 2.5 to 3.5.

7. A hair treatment emulsion as claimed in any of claims 4 to 6, characterized in that the emulsion contains the phospholipid and the inorganic and/or organic acid in a ratio by weight of from 2:1 to 1:2.

8. A hair treatment emulsion as claimed in any of claims 4 to 7, characterized in that the phospholipid is a lecithin, more especially egg lecithin or soya lecithin, and/or a kephalin.

9. A hair treatment emulsion as claimed in any of claims 4 to 8, characterized in that the inorganic acid is phosphoric acid, sulfuric acid and/or hydrochloric acid.

10. A hair treatment emulsion as claimed in any of claims 4 to 8, characterized in that the organic acid is citric acid or ascorbic acid.

11. A hair treatment emulsion as claimed in claim 10, characterized in that the additional emulsifier is cholic acid, lithocholic acid, deoxycholic acid, taurocholic acid, tannic acid, abietic acid, more especially in the form of their alkali metal or ammonium salts, and/or a saponin.

12. A hair treatment emulsion as claimed in any of claims 4 to 11, characterized in that the additional emulsifier is a salt of cholic acid, more especially the potassium salt of cholic acid.

13. A hair treatment emulsion as claimed in any of claims 4 to 12, characterized in that the emulsion contains from 0.1 to 10% by weight and preferably from 0.5 to 5% by weight of thickener and has a creamy consistency.

14. A hair treatment emulsion as claimed in claim 13, characterized in that the emulsion contains polysaccharides, particularly xanthan gum, guar gum, agar agar, alginates, carob bean flour and/or pectins as thickeners.

15. A hair treatment emulsion as claimed in any of claims 4 to 14, characterized in that the emulsion contains additional preservatives.

16. A hair treatment emulsion as claimed in claim 15, characterized in that the additional preservatives are selected from salicylic acid, formic acid, propionic acid, benzoic acid, sorbic acid, cinnamic acid, menthol, thymol, eugenol and lemon grass extract, particular significance being attributed to benzoic acid.

## Revendications

1. Utilisation d'un mélange de phospholipides d'origine naturelle avec des acides minéraux et/ou organiques en tant que combinaison de principes actifs dans une émulsion aqueuse acide pour le traitement des cheveux, en vue de l'amélioration de l'aptitude au peignage à l'état sec et à l'état humide des cheveux et en vue de la réduction de leur aptitude à la charge électrostatique, caractérisée en ce que :
a) les acides organiques sont choisis dans le groupe qui comprend l'acide acétique, l'acide lactique, l'acide tartrique, l'acide citrique, l'acide malique, l'acide ascorbique et l'acide gluconique, et
b) l'émulsion possède une valeur de pH allant de 2 à 3,5.

2. Utilisation selon la revendication 1 dans une émulsion aqueuse qui renferme des agents émulsionnants additionnels, de préférence d'origine naturelle.

3. Utilisation selon l'une des revendications 1 ou 2, dans une émulsion aqueuse qui possède une valeur de pH allant de 2,5 à 3,5,

4. Emulsion appropriée en vue de l'utilisation selon l'une des revendications 1 à 3, pour le traitement des cheveux, contenant un mélange de phospholipides d'origine naturelle avec des acides minéraux et/ou organiques en tant que combinaison de principes actifs ainsi que d'agents émulsionnants additionnels caractérisé en ce que :
a) les acides organiques sont sélectionnés dans le groupe qui comprend l'acide acétique, l'acide lactique, l'acide tartrique, l'acide citrique, l'acide malique, l'acide ascorbique et l'acide gluconique, et
b) l'émulsion possède une valeur de pH allant de 2 à 3,5.

5. Emulsion pour traitement des cheveux selon la revendication 4, caractérisée en ce que l'émulsion contient :
- de 0,1 à 20 % en poids - de préférence de 1 à 3 % en poids - d'un phospholipide d'origine naturelle,
- de 0,1 à 20 % en poids - de préférence de 1 à 3 % en poids - d'un acide minéral et/ou d'un acide organique,
- ainsi que
- de 0,01 à 2 % en poids - de préférence de 0,05 à 0,5 % en poids, d'un agent émulsionnant additionnel, de préférence d'origine naturelle.

6. Emulsion pour le traitement des cheveux selon l'une des revendications 4 ou 5, caractérisée en ce que l'émulsion possède une valeur de pH allant de 2,5 à 3,5.

7. Emulsion pour le traitement des cheveux selon l'une des revendications 4 à 6, caractérisée en ce que l'émulsion contient le phospholipide et l'acide minéral et/ou organique dans un rapport pondéral allant de 2 : 1 à 1 : 2.

8. Emulsion pour le traitement des cheveux selon l'une des revendications 4 à 7, caractérisée en ce que le phospholipide est une lecithine, en particulier de la lecithine d'oeuf ou de la lecithine de soja et/ou une céphaline.

9. Emulsion pour le traitement des cheveux selon l'une des revendications 4 à 8, caractérisée en ce que l'acide minéral est l'acide phosphorique, l'acide sulfurique et/ou l'acide chlorhydrique.

10. Emulsion pour le traitement des cheveux selon l'une des revendications 4 à 8, caractérisée en ce que l'on utilise comme acide organique de l'acide citrique ou de l'acide ascorbique.

11. Emulsion pour le traitement des cheveux selon l'une des revendications 4 à 10, caractérisée en ce que l'agent émulsionnant additionnel est l'acide cholique, l'acide lithocholique, l'acide desoxycholique, l'acide taurocholique, l'acide tannique, l'acide abiétique en particulier sous forme de leurs sels de métal alcalin ou d'ammonium, et/ou une saponine.

12. Emulsion pour le traitement des cheveux selon l'une des revendications 4 à 11, caractérisée en ce que l'agent émulsionnant additionnel est un sel d'acide cholique, en particulier le sel de potassium d'acide cholique.

13. Emulsion pour le traitement des cheveux selon l'une des revendications 4 à 12, caractérisée en ce que l'émulsion contient de 0,01 à 10 % en poids - de préférence de 0,5 à 5 % en poids - d'un agent épaississant et possède pour cela un état crémeux.

14. Emulsion pour le traitement des cheveux selon la revendication 13, caractérisée en ce que l'émulsion contient comme agent épaississant des polysaccharides, en particulier de la gomme xanthanne, de la gomme guar, de l'agar-agar, des alginates, de la farine de noyau de caroube, et/ou de la pectine.

15. Emulsion pour le traitement des cheveux selon l'une des revendications 4 à 14, caractérisée en ce que l'émulsion contient des agents de conservation additionnels.

16. Emulsion pour le traitement des cheveux selon la revendication 15, caractérisée en ce que les agents de conservation additionnels sont sélectionnés parmi l'acide salicylique, l'acide formique, l'acide propionique, l'acide benzoïque, l'acide sorbique, l'acide cinnamique, le menthol, le thymol, l'eugenol et l'extrait de Lemon Grass, parmi lesquels une importance particulière revient à l'acide benzoïque.
